# EUROPEAN PATENT APPLICATION

(11) **EP 4 220 172 A1**
(43) Date of publication of application: **02.08.2023**
(21) Application number: 22153733.5
(22) Date of filing: 27.01.2022
(51) Int. Cl.: G01N 33/68

(54) **COVID-19 THERANOSTIC**

(71) Applicant: Université Libre de Bruxelles, 1050 Bruxelles (BE)
(72) Inventor: De Vos, Nathalie, 2600 Berchem (BE)
(74) Representative: Calysta NV

(57) **Abstract**

A diagnostic based on Angiotensin 1-7 level, possibly in synergy with other blood parameters, to predict the outcome of infected patients by respiratory viral diseases and the corresponding drugs to prevent worsening of the disease for the patients predicted to be at higher risk.

## Description

### Background

The present invention is in the field of diagnostic associated to pathologies affecting the angiotensin signaling pathway, including viral respiratory diseases, and of companion drugs for the patients predicted to be at higher risk.

### Prior art

Angiotensin is a peptide hormone involved, among others in the blood pressure regulation and inflammatory processes. In this signaling pathway, angiotensinogen is converted into different smaller peptide hormones, including Angiotensin II (Ang-II) and Angiotensin 1-7 (Ang-(1-7)). Ang-(1-7) harbors anti-inflammatory properties, counter-acting several effects of Ang-II. Under the classical pathway, Ang-II is converted into Ang-(1-7) by the enzyme Angiotensin Converting enzyme 2 (ACE2), pointing to a finely regulated mechanism. Ang-(1-7) is also produced non-classically from angiotensinogen.

As for the first SARS coronavirus, the SARS-Cov-2 (COVID19) is known to target the ACE2, which is postulated to imbalance the Ang-II/Ang-(1-7) regulation, causing deleterious effects to the patient.

Several references disclose accordingly decreased blood levels of Ang-(1-7), upon SARS infection, including the SARS-CoV-2 (Covid-19) infection. Among them, Henry et al., 2021, CORONAVIRUS DISEASE 2019 is ASSOCIATED WITH LOW CIRCULATING PLASMA LEVELS OF ANGIOTENSIN 1 AND ANGIOTENSIN 1,7 DOI: 10.1002/jmv.26479. Interestingly, this reference suggests that the patients at the intensive care units have an even lower Ang-(1-7) blood level, as compared to patients less severely affected. 43 COVID positive patients have been enrolled, from which 13 were taking inhibitors of ACE (ACEi), or angiotensin receptor blockers (ARB), and the analysis performed on these patients have not been shown. The main comorbidities were diabetes (30.7%) and hypertension (30.7%). 17 patients were hospitalized and 7 required ICU. Ang-(1-7) has been measured by ELISA. No information has been given on how the blood samples have been treated. The levels of the upstream Angiotensin I are very strongly decreased in Covid-patients, as compared to healthy patients.

On the other hand, Valle Martins et al., 2021, INCREASED CIRCULATING LEVELS OF ANGIOTENSIN-(1-7) IN SEVERELY ILL COVID-19 PATIENTS, discloses opposite results. In this study, arterial blood samples of 19 severe COVID patients and of 19 non-COVID patients or healthy volunteers have been obtained and mixed with a denaturation solution to inactivate plasma proteases and Ang-(1-7) levels have been measured by mass spectrometry, these authors considering that ELISA does not well discriminate between Angiotensin 2 and Ang-(1-7). Several patients were on ACEi or ARB.

Such different results may origin from the different patient populations and different measurement methods of Ang-(1-7), whereas this peptide is known to be difficult to measure. Furthermore, due to the relationships between the different peptide hormones, where Ang-II is the substrate for ACE2 in the Ang-(1-7) production, but also where Ang-(1-7) can be produced independently of Ang-II and/or ACE2, as well as the major effect of the associated diseases, especially hypertension, or their treatment, on the level of these peptide hormones, the underlying situation is not clear and, from the prior art, the inventors conclude that the patients cannot be simply divided into two groups, depending on the level of Ang-(1-7).

### Summary of the invention

A first aspect of the invention is a method to predict whether a patient infected by a viral respiratory disease (SARS) is at risk of developing clinical complications, comprising the steps of:
- measuring the Angiotensin (1-7) level (Ang-(1-7) levels) from a blood sample of the patient at admission, preferably of TGFβ, CRP and/or ferritin as well, and of
- comparing the level measured in the blood sample with (i) the level found in patients infected by the viral respiratory disease and known to have a favorable outcome and with (ii) the level found in patients infected by the viral respiratory disease and known to have a poor outcome, wherein the viral respiratory disease is a SARS infection, preferably a SARS Cov-2 (COVID-19) infection.

A corresponding aspect of the present invention is the use of Ang-(1-7) agonists for the patients infected with a SARS virus and (ii) having a lower Ang-(1-7) levels.

Another corresponding related aspect is the use of antifibrotics or of anti-Spike antibodies for the patients infected with a SARS virus and (ii) having (a lower Ang-(1-7) levels or for the patients infected with a SARS virus and) having higher TGFβ and/or ferritin and or CRP levels.

### Brief description of the Figures

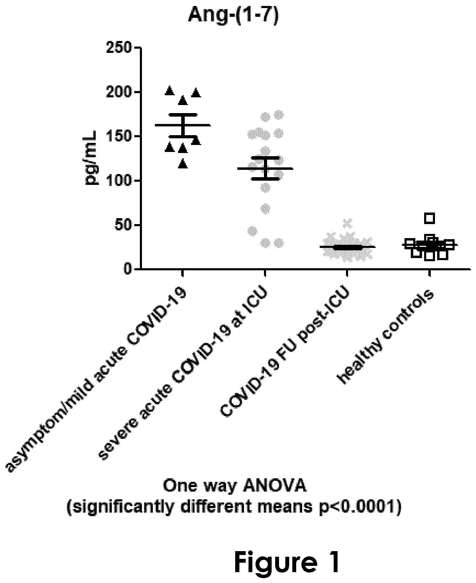

Figure 1 depicts Ang-(1-7) blood levels in different subgroup of patients.

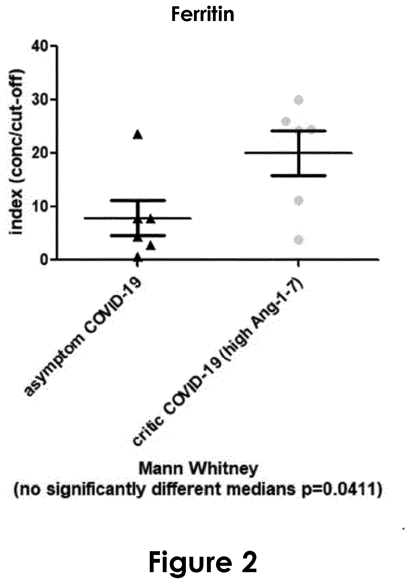

Figure 2 depicts ferritin blood levels in different subgroup of patients.

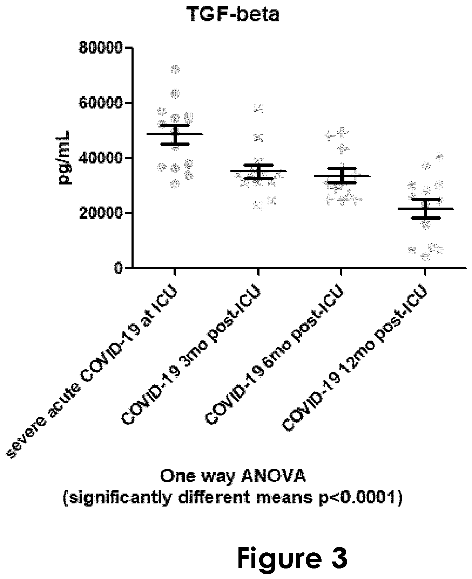

Figure 3 depicts TGFβ blood levels in different subgroup of patients.

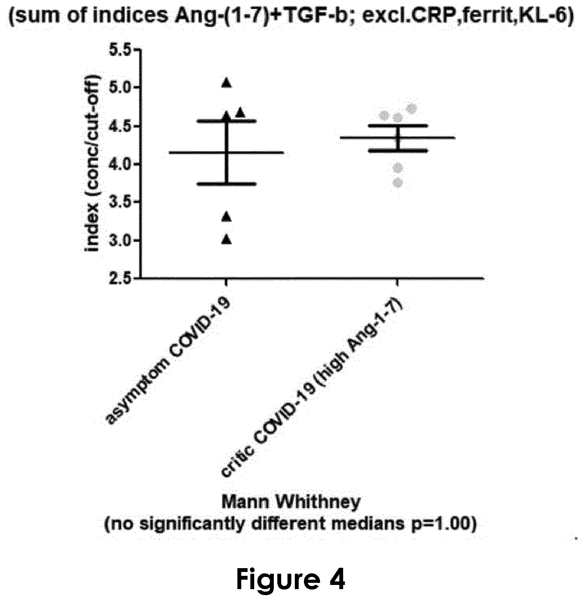

Figure 4 depicts a composite index with TGFβ and Ang-(1-7) blood levels in different subgroup of patients.

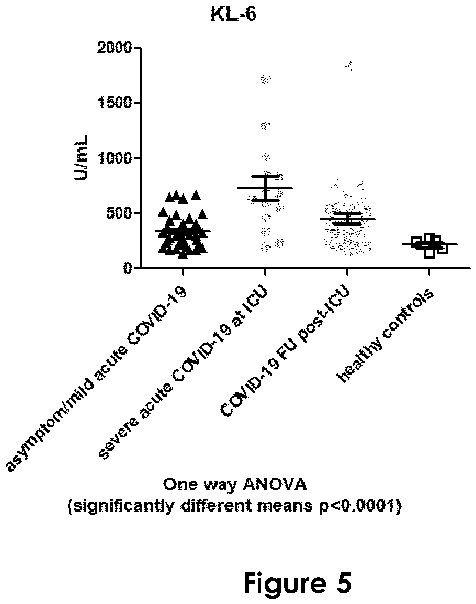

Figure 5 depicts KL-6 blood levels in different subgroup of patients.

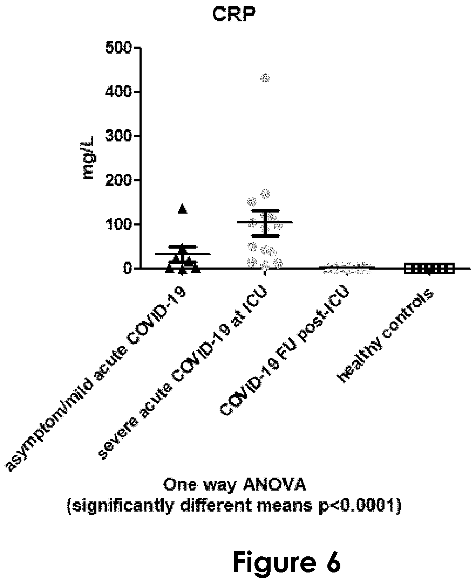

Figure 6 depicts CRP blood levels in different subgroup of patients.

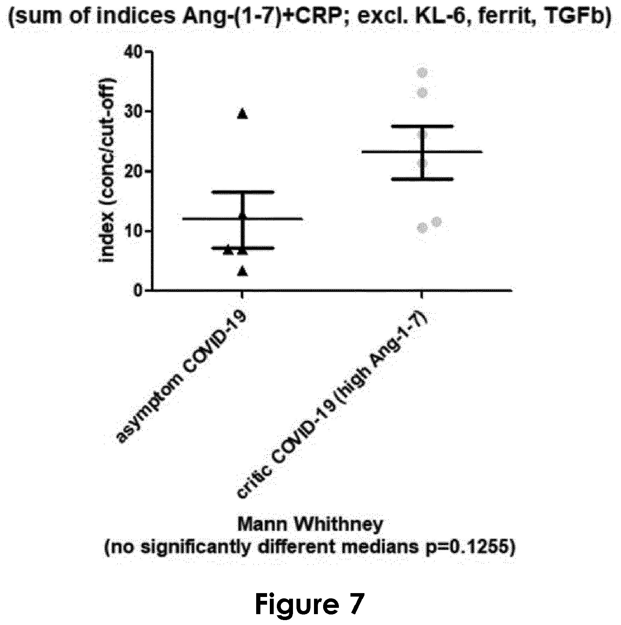

Figure 7 depicts a composite index with CRP and Ang-(1-7) blood levels in different subgroup of patients.

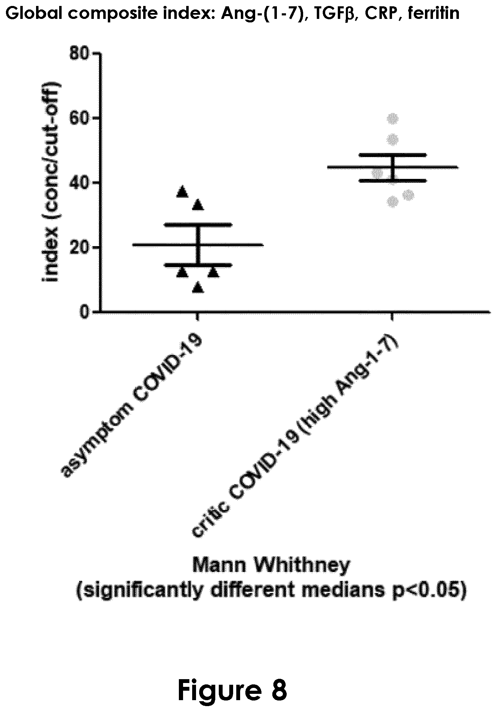

Figure 8 depicts a composite index with Ang-(1-7), TGFβ, ferritin and CRP levels.

### Detailed description of the invention

The inventors have measured several parameters from hospitalized patients. Among them, the blood level of the Angiotensin 1-7 (Ang-(1-7)) peptide (H-Asp-Arg-Val-Tyr-Ile-His-Pro-OH) has been found to be of paramount importance, even if the blood level of the peptide is difficult to precisely measure and does not allow a simple binary diagnostic. Indeed, the inventors have found that SARS (Covid19) infection results into an increased level of Ang-(1-7), which is paradoxical. However, a part of the patients has a lower increase, and these patients are at risk of further severe complications. Moreover, the inventors have found that a part of the patients with a high level of Ang-(1-7) are also at risk, and this risk can be predicted by measuring additional blood parameters. The risk, when correctly predicted, allows to rapidly provide the most appropriate treatment to the patients: mild treatments, Ang-(1-7) agonists, antifibrotics or monoclonal antibodies targeting the Spike protein of the virus, or oxygen supplementation. The correct diagnostic disclosed in the present invention is also valuable for allowing a better design of the clinical trials, where molecules are tested on the right subgroup of patients.

One problem with Ang-(1-7) measurement is its poor stability. To address this, the inventors have immediately put (venous) blood samples in ice-chilled tubes comprising EDTA, then immediately performed a cold centrifugation before storage at -80°C. Alternatively, the (venous) blood sample have been harvested in heparin-coated tubes before centrifugation and cold storage: 2-8°C for a few days, or at -80°C for longer periods.

Hence a first aspect of the invention is a method to predict whether a patient infected by a viral respiratory disease is at risk of developing clinical complications, comprising the steps of:
- (specifically) measuring the Ang-(1-7) level from a (venous) blood sample of the said patient at admission and of
- comparing the level measured in the said (venous) blood sample with (i) the level found in control patients and/or in patients infected by the said viral respiratory disease and known to have a favorable outcome and with (ii) the level found in patients infected by the said viral respiratory disease and known to have a poor outcome
wherein the viral respiratory disease is preferably a SARS, preferably a SARS2 infection, preferably a SARS COVID-19 infection.

Preferably, the (venous) blood sample has been obtained and kept in conditions to avoid Ang-(1-7) degradation, including advantageously, storage at temperature close to 0°C or below.

Preferably, in this method, the Ang-(1-7) level in the patient is higher than the Ang-(1-7) level in healthy subjects and/or not infected by the SARS infection (SARS2; SARS COVID-19 infection). The inventors have found that such a control ensures an absence of bias in the measurement step of Ang-(1-7), such as possibly the measurement of Angiotensin-2 together with Ang-(1-7).

Preferably this method is further comprising the step of measuring the level of KL-6 and/or of TGFβ in a blood sample from the patient (the same blood sample or another blood sample). The amount of these factors, when increased, is associated to a worse diagnostic, including in patients having a favorable Ang-(1-7) level.

Preferably this method is further comprising the step of measuring the level of the C reactive protein (CRP) and/or of ferritin in a blood sample from the patient. The inventors have found that these markers are associated with an adverse immune response; hence synergize with the above markers, especially the Ang-(1-7) measurement.

Advantageously, at least Ang-(1-7), CRP, ferritin and TGFβ are measured on the blood sample at admission.

Preferably, during the follow-up period of the patient, TGFβ and/or KL-6 levels are measured in a blood sample of the patient. This allows to follow the outcome of the disease and, if these markers are elevated, to provide antifibrotics to the patient.

Preferably, in this method, the Ang-(1-7) level has been measured using the same tools on (i) at least 10 (preferably at least 15, 20, 25, 30, 40, 50) patients with a favorable outcome and (ii) on at least 10 (preferably at least 15, 20, 25, 30, 40, 50) patients with a poor outcome and, possibly at least 5 (preferably at least 10, 15, 20, 25, 30, 40, 50) healthy person. This allows to stratify the patients; hence establishing a limit for the Ang-(1-7) level associated with a poor outcome. This stratification is preferably also implemented for the other markers: KL-6, TGFβ, CRP, ferritin.

Alternatively, the levels are compared to control values.

Preferably, in the method, the measurement of Ang-(1-7) and possibly of the other markers, such as KL-6, CRP, ferritin and/or TGFβ are done using immunoassays, (immuno-)turbidimetry, nephelometry, spectrophotometry, (capillary) electrophoresis, densitometry, immunofixation, iso-electric focusing, chromatographic techniques, mass spectrometry (including but not limited to LC-MS, GC-MS, MS/MS, MALDI-TOF), atomic emission (flame photometry), atomic absorption spectrophotometry, refractometry, electrochemistry (potentiometry, coulometry, amperometry), molecular luminescence spectroscopy (fluorometry), lab-on-a-chip, biosensor technology containing a transducer with electrochemical properties (potentiometric, amperometric, or conductometric), mass (piezoelectric), heat (calorimetric), or optical properties (luminescent, fluorescent, reflective). Preferably, in this method, the measurements (of Ang-(1-7), KL-6, TGFβ, CRP and/or of ferritin) are done using immunoassays, such as Enzyme-Linked Immunosorbent Assay (ELISA), and/or by ChemiLuminescence Enzyme ImmunoAssay (CLEIA), which can advantageously be achieved using the Lumipulse^{®} platform, Electro-chemiluminescence Immunoassay (ECLIA), Chemiluminescent immunoassay (CLIA), enzyme immunoassay (EIA), radioimmunoassay (RIA), immunochromatographic strip. The immunoassays can also be multiplexed or singleplexed in arrays and/or beads, which can be magnetic.

The antibody used in the immunoassay is monoclonal and specific for Ang-(1-7), and substantially does not cross-react with the related other angiotensin peptides, especially Angiotensin II.

In the context of the present invention, the terminology "substantially does not cross react with other angiotensin peptides (Angiotensin II)" preferably means that the affinity of the antibody for Ang-(1-7) is at least 10-fold higher than the affinity for another angiotensin peptide (Angiotensin II), preferably at least 30-fold higher, more preferably at least 100-fold higher. Conversely, this terminology "substantially does not cross react with other angiotensin peptides (Angiotensin II)" preferably means that at least 50% of the signal of the immunoassay is specific for Ang-(1-7) and less than 50% of the signal is associated to other forms of the Angiotensin peptide (including Ang II), preferably at least 60%, at least 70%, at least 75%, at least 80%, at least 90% or even at least 95% of the signal is specific for Ang-(1-7), the remaining part being associated to other forms of the Angiotensin peptide (including Ang II).

The specificity can be tested for instance by mixing known amounts of the Ang-(1-7) peptide and of other angiotensin peptides (including Ang II).

The advantages of the immunoassays are the easiness of implementation, allowing large-scale routine measurement, as well as robust and reliable values.

As above-mentioned, other measurement methods can advantageously be used, either in routine practice or for the validation of the above immunoassay values, including chromatographic techniques and mass spectrometry. The advantage of mass-spectrometry or of chromatographic techniques over the immunoassays is related to a more robust readout, with less risk of cross-reactivity-induced false signal.

A related aspect of the present invention is the correction of the identified deficit in Ang-(1-7) in a subgroup of patients affected by the viral disease (SARS; SARS Cov2; Covid-19).

Hence the present invention relates to Angiotensin 1-7 agonists for use in patients having a blood level of Ang-(1-7) below (i) the level found in patients infected by the viral respiratory disease and known to have a favorable outcome and/or comparable with (ii) the level found in patients infected by the said viral respiratory disease and known to have a poor outcome.

The preferred Ang-(1-7) agonists are selected from the group consisting of Angiotensin 1-7 (the heptapeptide itself; for instance formulated as TXA127, or Ang-(1-7) derived from plasma), Ang-(1-7) analogs, including the molecules defined in EP2967049, recombinant (soluble) hACE2 including APN01, Biophytis BIO0101 (also known as Sarconeos; a MasR agonist), Angiotensin II receptor type 2 (AT2R) agonists (C21 or VP01).

In-line with the above-method, the KL-6 and/or TGFβ level(s) in the patient to be given the Ang(1-7) agonist is (are) preferably higher than (i) the level found in patients infected by the said viral respiratory disease and known to have a favorable outcome, and preferably, the CRP and/or the ferritin level(s) is (are) higher than (i) the level found in patients infected by the viral respiratory disease and known to have a favorable outcome.

In other words, this aspect corresponds to a method of treatments of patients infected by a respiratory disease (SARS2: SARS-Cov19), the method comprising the steps of
- measuring the Angiotensin-(1-7); Ang-(1-7) level from a blood sample of the said patient at admission and of
- comparing the level measured in the said blood sample with (i) the level found in patients infected by the said viral respiratory disease and known to have a favorable outcome and/or with (ii) the level found in patients infected by the said viral respiratory disease and known to have a poor outcome
wherein the Ang-(1-7) agonists are administered to the patients having Ang(1-7) level below (i) (mentioned above) or corresponding to (ii) (mentioned above).

Preferably, this method of treatment further comprises to perform the diagnostic as above as a preliminary step.

Preferred angiotensin (1-7) agonists are selected from the group consisting of Angiotensin 1-7 (the heptapeptide itself; for instance formulated as TXA127, or Ang-(1-7) derived from plasma), Ang-(1-7) analogs, including the molecules defined in EP2967049, recombinant (soluble) hACE2 including APN01, Biophytis BIO0101 (also known as Sarconeos; a MasR agonist), Angiotensin II receptor type 2 (AT2R) agonists (C21 or VP01).

A corresponding related aspect is one or several antifibrotics for use in patients being infected by a viral respiratory disease (SARS) and having either an Angiotensin 1-7 level in the patient lower than (i) the level found in patients infected by the said viral respiratory disease and known to have a favorable outcome, or having an Angiotensin 1-7 level corresponding to (i) the level found in patients infected by the said viral respiratory disease and known to have a favorable outcome, but a KL-6 and/or TGFβ level found higher than (i) the level found in patients infected by the said viral respiratory disease and known to have a favorable outcome, and preferably, wherein the CRP and/or the ferritin level(s) is (are) higher than (i) the level found in patients infected by the said viral respiratory disease and known to have a favorable outcome.

Alternatively, the antifibrotic(s) is for use in patients being infected by a viral respiratory disease (SARS; SARS-Cov2) and having increased TGFβ, CRP (preferably KL-6 as well) and ferritin levels as compared with healthy patients.

This is to treat patients having an impaired signaling downstream to Ang-(1-7) or an adverse immune response, as a consequence to the viral infection (SARS; SARS-Cov2).

In the context of the present invention, the preferred antifibrotics are selected from the group consisting of Nintedanib, Pirfenidone, Treamid, collagene-polyvinylpyrrolidone, glucocorticoids (e.g. Dexamethasone), corticosteroids (including Prednisone, methylprednisone, hydrocortisone) budesonide, Bovhyaluronidase azoximer, BIO 300 from Genistein (i.e. genistein nanosuspension), Tetrandrine, Fuzheng Huayu Tablet, Anluohuaxian, Stromal Vascular Fraction, IN01 Vaccine (EGF inhibitor), Interleukin-6 inhibitor (tocilizumab, sarilumab), antiviral treatments such as ritonavir + PF-07321332 (Paxlovid ^{®} ) and molnupiravir, SARS-Cov-2-neutralizing antibodies either plasma from convalescent patients or Spike antibodies; see below), mucolytica (e.g. N-acetylcystein) and antioxidants (e.g. vitamin A and/or beta carotene; Vitamin C; Vitamin D and/or alfacalcidol, clcifediol, calcidiol, calcitriol, cholecalciferol; Vitamin E and/or tocopherol; selenium, zinc).

In other words, this aspect corresponds to a method of treatments of patients infected by a respiratory disease (SARS2: SARS-Cov19), the method comprising the steps of :
- measuring the Angiotensin (1-7); Ang(1-7) level, and KL-6 and/or TGFβ and/or CRP and/or ferritin level(s) from a blood sample of the said patient at admission and of
- comparing the level measured in the said blood sample with (i) the level found in patients infected by the said viral respiratory disease and known to have a favorable outcome and with (ii) the level found in patients infected by the said viral respiratory disease and known to have a poor outcome,
wherein the antifibrotics are administered to the patients having KL-6 and/or TGFβ and/or CRP and/or ferritin levels higher than, or equal to, the level corresponding to (ii) above.

Alternatively, the method of treatments of patients infected by a respiratory disease (SARS2: SARS-Cov19), comprises the steps of
- measuring TGFβ and/or CRP and/or ferritin level(s) from a (venous) blood sample of the said patient at admission and of
- comparing the level measured in the said (venous) blood sample with (i) the level found in patients infected by the said viral respiratory disease and known to have a favorable outcome and with (ii) the level found in patients infected by the said viral respiratory disease and known to have a poor outcome;
wherein the antifibrotics are administered to the patients having or TGFβ and/or CRP and/or ferritin levels higher than, or equal to, the level corresponding to (ii) above.

In addition, the blood level of KL-6 is preferably measured and antifibrotics are provided to the patients with high KL-6 levels

Preferably, this method further comprises as preliminary steps the other steps of the diagnostic method as above.

This allows to prevent or reduce the complications caused by the SARS infection, including the adverse immune response and/or the pulmonary fibrosis.

Accordingly, another related aspect of the present invention is related to monoclonal antibodies specifically recognizing the spike protein for use in the patients predicted to be at risks by the diagnostic of the present invention.

The preferred Spike-specific antibodies are sotrovimab, tixagevimab, cilgavimab, casirivimab, imdevimab, bamlanivimab, etesevimab and mixtures thereof.

Similarly, the invention further relates to a treatment selected from:
- High-flow oxygen (oxygen by mask, oxygen by high-flow nasal cannula, non-invasive mechanical ventilation), mechanical ventilation, ECMO extracorporeal membrane oxygenation;
- Glucocorticoids
- interleukin-6 inhibitor (tocilizumab, sarilumab)
- Protein kinase inhibitors and JAK inhibitors (baricitinib, tofacitinib)
- Antiviral treatments like remdesivir, or SARS-CoV-2 protease inhibitors, e.g. per os ritonavir + PF-07321332 (Paxlovid ^{®}), molnupiravir,
- Mucolytica (N-acetylcystein),
- Antioxidantia (vitamin A and/or beta carotene; Vitamin C; Vitamin D and/or alfacalcidol, clcifediol, calcidiol, calcitriol, cholecalciferol; Vitamin E and/or tocopherol; selenium, zinc) and
- Low molecular weight heparin or unfractionated heparin for use in the patients determined to be at risk by the above diagnostic, or to a method of treatment of these patients.

### Examples.

Samples were prospectively drawn from 22 severe COVID-19 patients during their pneumonology follow-up (FU) at 3, 6 and 12 months post-ICU. Blood samples were obtained in heparine-coated tubes, then centrifuged and the plasma was kept in heparin-containing tubes and stored at temperatures between 2 and 8°C for a few days, or frozen (-80°C) for longer periods. Patients were compared with three groups consisting of 7 mild/asymptomatic COVID-19 patients, 5 healthy controls and 2 patients with bacterial pneumonia. Novel biomarkers were analyzed with Lumipulse^{®} G KL-6 assay (Fujirebio, Japan), Human ANG1-7 ELISA kit RUO (KyvoBio SRL, Belgium) and Human TGF-β1 Quantikine ELISA RUO (R&D Systems, USA). Routine biomarkers like C-reactive protein (CRP), ferritin, cardiac troponin T (cTnT) and lactate dehydrogenase (LDH) were tested on Cobas 8000 (Roche, Germany), and procalcitonin (PCT) was analyzed on Lumipulse^{®} G1200 (Fujirebio, Japan). Data are expressed as [median (IQR)]. P-values <0.05 were considered statistically significant. Cut-offs to distinguish between severe COVID-19 patients and healthy subjects were obtained with receiver operating curve (ROC). For each biomarker, the result divided by its cut-off, gave an index. The sum of the indices of Ang-(1-7), TGF- β1, CRP and ferritin was used to generate a composite index.

Patient characteristics were 73% men, mean age of 55, 50% obese, 45% arterial hypertension (HTA), 27% diabetes, 100% acute respiratory distress syndrome (ARDS), a mean length of stay (LOS) of 21d at ICU, 64% intubation and 9% extracorporeal membrane oxygenation (ECMO) during the acute SARS-CoV-2 infection.

At ICU, the severe COVID-19 patients showed significantly higher biomarkers KL-6 [665 U/mL (441-900)], Ang-(1-7) [124 pg/mL (81-154)], TGF-β [51204 pg/mL (36711-55959)], PCT [0.30 µg/L (0.06-1.20)], CRP [97 mg/L (32-132)], ferritin [539 µg/L (133-1504)], cTnT [20.15 ng/L (10.93-34.38)] and LDH [503 U/L (404-747)] compared to healthy controls (p<0.05). Within the population of severe acute COVID-19 at ICU, two subgroups were found, one with Ang-(1-7) deficit and another subgroup with high Ang-(1-7) that could be distinguished from the asymptomatic COVID-19 thanks to the present diagnostic method (p<0.05). KL-6, Ang-(1-7) and CRP marked the severity, as they were higher in severe COVID-19 patients compared to asymptomatic/mild COVID-19 cases (p<0.05). The inventors have found that, besides Ang-(1-7), CRP, ferritin and TGFβ levels were correctly discriminating a part of the populations, with median values significantly different, but their combination with Ang-(1-7) was needed to develop a robust global comprehensive prediction test discriminating all the at-risk patients from the patients with a favorable outcome, and discriminating a patient subgroup where Ang(1-7) supplementation will be beneficial.

## Claims

1. A method to predict whether a patient infected by a viral respiratory disease is at risk of developing clinical complications, comprising the steps of:
- measuring the Angiotensin-(1-7) level from a blood sample of the said patient at admission and
- comparing the level measured in the said blood sample with (i) the level found in patients infected by the said viral respiratory disease and known to have a favorable outcome and with (ii) the level found in patients infected by the said viral respiratory disease and known to have a poor outcome,
wherein the said viral respiratory disease is a SARS infection, preferably a SARS Cov-2 (COVID-19) infection.

2. The method of claim 1, wherein the Angiotensin (1-7) level in the patient is higher than the Angiotensin (1-7) level in healthy subjects not infected by the SARS infection, preferably by the SARS Cov-2 (COVID-19) infection.

3. The method of claim 1 or 2, further comprising the step of measuring the level of KL-6 in a blood sample from the said patient.

4. The method according to any one of the preceding claims, further comprising the step of measuring the level of TGFβ in a blood sample from the said patient.

5. The method according to any one of the preceding claims, further comprising the step of measuring the level of the C reactive protein (CRP) and/or of ferritin in a blood sample of the said patient.

6. The method according to any one of the preceding claims, wherein the Angiotensin (1-7) level has been measured using the same tools on (i) at least 10 (preferably at least 15, 20, 25, 30, 40, 50) patients with a favorable outcome and (ii) on at least 10 (preferably at least 15, 20, 25, 30, 40, 50) patients with a poor outcome and, possibly at least 5 (preferably at least 10, 15, 20, 25, 30, 40, 50) healthy person.

7. The method according to any one of the preceding claims, wherein the measurements are done using immunoassays, (immuno-)turbidimetry, nephelometry, spectrophotometry, electrophoresis, densitometry, immunofixation, iso-electric focusing, chromatographic techniques, mass spectrometry, atomic emission, atomic absorption spectrophotometry, refractometry, electrochemistry, molecular luminescence spectroscopy, by lab-on-a-chip, using biosensor technology containing a transducer with electrochemical properties, by mass, heat, or optical properties measurement.

8. The method of claim 7, wherein the immunoassays are selected from the group consisting of Enzyme-Linked Immunosorbent Assay (ELISA), ChemiLuminescence Enzyme ImmunoAssay (CLEIA), Electro-chemiluminescence Immunoassay (ECLIA), Chemiluminescent immunoassay (CLIA), enzyme immunoassay (EIA), radioimmunoassay (RIA), immunochromatographic strip, biochip (multi-)array technology, bead-based multi- and/or singleplex (immuno-)assay, preferably wherein a monoclonal antibody specific for Angiotensin (1-7) is used in the said bioassay and the said antibody substantially does not cross-react with angiotensin II.

9. Angiotensin (1-7) agonists for use in patients according to any one of the preceding claims, wherein the Angiotensin (1-7) level in the patient is lower than (i) the level found in patients infected by the said viral respiratory disease and known to have a favorable outcome.

10. The angiotensin (1-7) agonists of claim 9, being selected from the group consisting of Angiotensin (1-7), the molecules defined in EP2967049, recombinant (soluble) hACE2, Biophytis BIO0101, TXA127, Angiotensin II receptor type 2 (AT2R) agonists.

11. The angiotensin (1-7) agonists of claim 9 or 10, wherein the KL-6 and/or TGFβ level(s) in the patient is (are) higher than (i) the level found in patients infected by the said viral respiratory disease and known to have a favorable outcome, and preferably, wherein the CRP and/or the ferritin level(s) is (are) higher than (i) the level found in patients infected by the said viral respiratory disease and known to have a favorable outcome.

12. Antifibrotics for use in patients according to any one of the preceding claims 1 to 8, the said patients being infected by a viral respiratory disease and having either
an Angiotensin (1 -7) level in the patient lower than (i) the level found in patients infected by the said viral respiratory disease and known to have a favorable outcome, or having
an Angiotensin (1-7) level corresponding to (i) the level found in patients infected by the said viral respiratory disease and known to have a favorable outcome, but a TGFβ and/or a CRP and/or a ferritin level(s) higher than (i) the level found in patients infected by the said viral respiratory disease and known to have a favorable outcome, or having
a TGFβ and a CRP and a ferritin level higher than (i) the level found in patients infected by the said viral respiratory disease and known to have a favorable outcome.

13. The antifibrotics of claim 12, being selected from the group consisting of Nintedanib, Pirfenidone, Treamid, collagene-polyvinylpyrrolidone, glucocorticoids, corticosteroids, budesonide, Bovhyaluronidase azoximer, Genistein, Tetrandrine, Fuzheng Huayu Tablet, Anluohuaxian, Stromal Vascular Fraction, IN01 Vaccine.

14. Spike-specific monoclonal antibodies for use in patients according to any one of the preceding claims 1 to 8, the said patients being infected by a viral respiratory disease and having either an Angiotensin 1-7 level in the patient lower than (i) the level found in patients infected by the said viral respiratory disease and known to have a favorable outcome, or having
an Angiotensin (1-7) level corresponding to (i) the level found in patients infected by the said viral respiratory disease and known to have a favorable outcome, but a TGFβ and/or a CRP and/or a ferritin level(s) higher than (i) the level found in patients infected by the said viral respiratory disease and known to have a favorable outcome, or having
a TGFβ and a CRP and a ferritin level higher than (i) the level found in patients infected by the said viral respiratory disease and known to have a favorable outcome.

15. The spike-specific antibodies of claim 14 being selected from the group consisting of sotrovimab, tixagevimab, cilgavimab, casirivimab, imdevimab, bamlanivimab, etesevimab and mixtures thereof.
